# EUROPEAN PATENT SPECIFICATION

(11) **EP 3 782 692 B1**
(45) Date of publication and mention of the grant of the patent: **25.02.2026**
(21) Application number: 19193408.2
(22) Date of filing: 23.08.2019
(51) Int. Cl.: A61M 39/06

(54) **HEMOSTASIS VALVE ASSEMBLY AND METHOD FOR ASSEMBLING A HEMOSTASIS VALVE**
HÄMOSTASEVENTILANORDNUNG UND VERFAHREN ZUR MONTAGE EINES HÄMOSTASEVENTILS
ENSEMBLE DE VALVE HÉMOSTATIQUE ET PROCÉDÉ D'ASSEMBLAGE D'UNE VALVE HÉMOSTATIQUE

(43) Date of publication of application: 24.02.2021
(73) Proprietor: Creganna Unlimited Company, Ballybrit, Galway H91 VN2T (IE)
(72) Inventor: MULDOON, Damian, Galway, H62 KPO3 (IE); MURPHY, Brian, Galway, H91V8XA (IE); WARD, Shane, Galway (IE); RUDDY, Liam, Mayo (IE)
(74) Representative: Grünecker Patent- und Rechtsanwälte PartG mbB

(56) References cited:
- EP-A2- 0 875 262
- EP-B1- 0 875 262
- WO-A1-01/17587
- WO-A1-2010/087943
- WO-A1-99/45996
- US-A- 4 798 594
- US-A- 4 929 235
- US-A- 5 009 391
- US-A- 5 167 637
- US-A- 5 643 227
- US-A1- 2004 230 161
- US-A1- 2010 179 480
- US-B2- 9 398 923

## Description

The present invention relates to a hemostasis valve assembly and to a method for assembling a hemostasis valve assembly, for instance for a structural heart catheter based delivery system, which is intended for delivering an intravascular device to targeted anatomy within the heart such as at the mitral annulus.

Intravascular medical procedures allow the performance of therapeutic treatments in a variety of locations within a patient's body while requiring only relatively small access incisions. An intravascular procedure may, for example, eliminate the need for open-heart surgery, reducing risks, costs, and time associated with an open-heart procedure. The intravascular procedure also enables faster recovery times with lower associated costs and risks of complication. An example of an intravascular procedure that significantly reduces procedure and recovery time and cost over conventional open surgery is a heart valve replacement or repair procedure in which an artificial valve or valve repair device is guided to the heart through the patient's vasculature. For example, a catheter is inserted into the patient's vasculature and directed to the inferior vena cava. The catheter is then urged through the inferior vena cava toward the heart by applying force longitudinally to the catheter. Upon entering the heart from the inferior vena cava, the catheter enters the right atrium. The distal end of the catheter may be deflected by one or more deflecting mechanisms, which can be achieved by a tension cable, or other mechanisms positioned inside the catheter.

However, when performing interventional cardiology, it is necessary to provide blood sealing during the insertion of medical material into the body of a patient. The medical material is for example a catheter, a guide wire, a sheath containing a valve or stent, or the like. To prevent leaks of bodily fluids, in particular blood, during the insertion of the material, it is known to use a hemostasis valve assembly at the insertion point into the patient. Such a hemostasis valve assembly comprises at least one valve for performing a sealing function around the inserted material and for being closed when idle.

Existing hemostasis valve assemblies, however, have the problem that each design is usable only with a small range of diameters of medical material. They also have the problem that for comparatively large diameters (e. g. 34 Fr, i. e. 11.3 mm) no cost efficient off the shelf solutions exist. Presently, the largest Fr size hemostasis valve assembly available as regular off the shelf component is 17 Fr (i. e. 5.7 mm). However, transcatheter aortic valve implantation (TAVI), transaortic mitral valve replacement (TAMVR), and other cardiological therapies require hemostasis valve arrangements with larger inner diameters.

There is still a need for an improved hemostasis valve assembly that has a consistent outer profile but can be adapted to have a wide range of inner profiles, further having a high degree of safety during use, at the same time being robust and economic to manufacture.

This object is solved by the subject matter of the independent claims. Advantageous embodiments of the present invention are the subject matter of the dependent claims.

The present invention is based on the idea that by providing a modular valve assembly with an outer housing and an end cap which can be used with all possible diameters of medical material combined with exchangeable components, namely a distal seal, a proximal seal, and a spacer element, a high design flexibility can be achieved. A large range of diameters of medical material can be covered easily at low cost.

In particular, the present invention provides a hemostasis valve assembly comprising a hollow body delimiting an inner passage for inserting a medical material extending between a proximal opening and a distal opening of the hollow body, a distal seal arranged at the distal opening of the hollow body and comprising an elastic valve, which is sealed when idle, a proximal seal arranged at the proximal opening of the hollow body, a spacer element, which is arranged between the distal seal and the proximal seal, and an end cap, wherein the end cap has fixing means engaging with support means provided at the hollow body, so that the stacked assembly comprising the distal seal, the spacer, and the proximal seal is fixed inside the hollow body.

This solution has the further advantage that each individual part can be fabricated separately, taking advantage of the cost-effectiveness of standardized large-scale production. Moreover, different materials can be used for the fabrication of the body, spacer element, and seals without the need of complex technologies, e. g. multi-material injection molding. Finally, low insertion and retraction forces can be achieved, which is advantageous for sensitive medical material.

According to an advantageous embodiment of the present invention, the fixing means is attached to the support means in a static manner. In particular, no threaded connection is used. This solution has the advantage that the assembly can be performed easily and reproducibly.

For instance, the fixing means may comprise at least one snap-fit protrusion, wherein the support means comprises at least one corresponding snap-fit recess into which the snap-fit protrusion engages. This a particularly cost efficient possibility to realize the fixing of the end cap at the hollow body. If desired, the fixing means may be attached in a detachable manner, so that the seals and/or the spacer element can be replaced.

According to the present invention, the distal seal comprises an elastic planar seal having a first radial slit extending through approximately half of the total axial thickness, and a second radial slit extending through the remaining half of the total axial thickness, wherein the first and the second slit are arranged to include a radial angle with each other. Thus, the two slits intersect in a very small, ideally point-shaped region in the center of the distal seal, so that the seal is closed when idle. Advantageously, the medical material usually comprises a thin guide wire which is inserted before placing the components having a larger diameter.

This thin guide wire is easily pushed through the intersection of the two slits, while the distal seal safely seals the passage around the guide wire. When the medical material with a larger diameter (e. g. a catheter) follows the guide wire, at least a part of the regions of the seal, which are weakened by the incisions, may be ruptured to allow the passage of the medical material. However, it could be shown that the sealing around the outer surface of the inserted medical material is very effective. Advantageously, the first and the second radial slit are orthogonal to each other.

Of course, also other than 90° angles can be provided and also more than two intersecting slits can be arranged in a way that at least one slit is cut from the first surface of the seal to at least half of the seal's thickness, while the other slits are cut from the opposing surface to at least half of the seal's thickness.

The distal seal may be a part common to all sizes of medical material to be inserted, or may also have optimized slit lengths and geometries to suit specific requirements e. g. of a catheter.

According to the present invention, the proximal seal comprises a ring shaped elastic planar seal with an inner opening delimiting a through passage aperture for receiving the medical material therethrough. Thus, an improved guidance and sealing of the medical material can be achieved. In particular, the inner opening of the proximal seal may be adapted to fit around the outer diameter of a particular type of catheter. Thus, the inserted catheter is supported and sealed by both the distal and the proximal seal, whereas the proximal seal does not represent an obstacle for the thinner guide wire.

In order to ensure a safe passage of the medical material through the spacer element, the inner opening of the proximal seal has a diameter which is smaller than an inner width of the spacer element.

The distal and/or the proximal seal are for instance fabricated from an elastic material, such as silicone.

According to an advantageous example of the present disclosure, the end cap has an essentially tubular shape and is at least partly encompassed by at least a part of the hollow body. This allows for a particularly accurate radial alignment of the individual parts of the modular hemostasis valve assembly.

Advantageously, the distal seal has a first peripheral region which is compressed between a retaining region at the distal opening of the hollow body and the spacer, wherein the proximal seal has a second peripheral region which is compressed between the spacer and the end cap, and wherein the axial compression forces are generated by the locking between the end cap and the hollow body. Thus, with one single locking mechanism, a secure clamping and accurate radial and axial alignment of both seals can be achieved.

The present invention further relates to a corresponding assembly method. In particular, a method for assembling a hemostasis valve comprises the following steps:
providing a hollow body delimiting an inner passage for inserting a medical material extending between a proximal opening and a distal opening of the hollow body,
in an axial direction, inserting a distal seal to be arranged at the distal opening of the hollow body, the distal seal comprising an elastic valve, which is sealed when idle,
inserting a spacer element to be in contact with the distal seal,
inserting a proximal seal to be arranged at the proximal opening of the hollow body, so that the spacer element is arranged between the distal seal and the proximal seal,
mounting an end cap, wherein the end cap has fixing means engaging with support means provided at the hollow body, so that the stacked assembly comprising the distal seal, the spacer, and the proximal seal is fixed inside the hollow body.

With such a modular assembly, each individual part can be fabricated separately, taking advantage of the cost-effectiveness of standardized large-scale production. Moreover, different materials can be used for the fabrication of the body, spacer element, and seals without the need of complex technologies, e. g. multi-material injection molding. A high design flexibility can be achieved and a large range of diameters of medical material can be covered easily at low cost and with high precision.

Advantageously, at least one of the hollow body, the spacer, and the end cap is manufactured by 3D printing. This technology allows for a cost-effective, customized fabrication which may also use specifically biocompatible materials.

A particularly fast, accurately aligned, and reliable connection between the hollow body and the end cap can be achieved when the end cap is mounted at the hollow body in a static manner by means of a snap-fit connection. Thus the end cap can be inserted into the hollow body in an axial direction without the need of any threaded parts. The axial forces holding together the assembly can be reached and maintained with improved reproducibility compared to threaded connections.

According to an advantageous example of the present disclosure, during the step of mounting the end cap, a first peripheral region of the distal seal is compressed between a retaining region at the distal opening of the hollow body and the spacer, and a second peripheral region of the proximal seal is compressed between the spacer and the end cap, and wherein the axial compression forces are maintained by the locking between the end cap and the hollow body. This allows for a secure clamping of the seals, at the same time providing improved radial and axial alignment of all components with respect to each other.

The accompanying drawings are incorporated into the specification and form a part of the specification to illustrate several embodiments of the present invention. These drawings, together with the description serve to explain the principles of the invention. The drawings are merely for the purpose of illustrating the preferred and alternative examples of how the invention can be made and used, and are not to be construed as limiting the invention to only the illustrated and described embodiments. Furthermore, several aspects of the embodiments may form-individually or in different combinations-solutions according to the present invention. The following described embodiments thus can be considered either alone or in an arbitrary combination thereof. Further features and advantages will become apparent from the following more particular description of the various embodiments of the invention, as illustrated in the accompanying drawings, in which like references refer to like elements, and wherein:
- **FIG. 1**: is a schematic partly exploded perspective representation of a hemostasis valve assembly according to the present invention;
- **FIG. 2**: is a schematic sectional view of the hemostasis valve assembly of Fig. 1 in a first operational state;
- **FIG. 3**: is a schematic sectional view of the hemostasis valve assembly of Fig. 1 in a second operational state;
- **FIG. 4**: is a schematic sectional view of the distal seal shown in Fig. 1;
- **FIG. 5**: is a further schematic sectional view of the distal seal shown in Fig. 1;
- **FIG. 6**: is a further schematic sectional view of the distal seal shown in Fig. 1;
- **FIG. 7**: is a further schematic sectional view of the distal seal shown in Fig. 1;
- **FIG. 8**: is a schematic sectional view of a further example of a hemostasis valve assembly;
- **FIG. 9**: is a schematic sectional view of a further example of a hemostasis valve assembly.

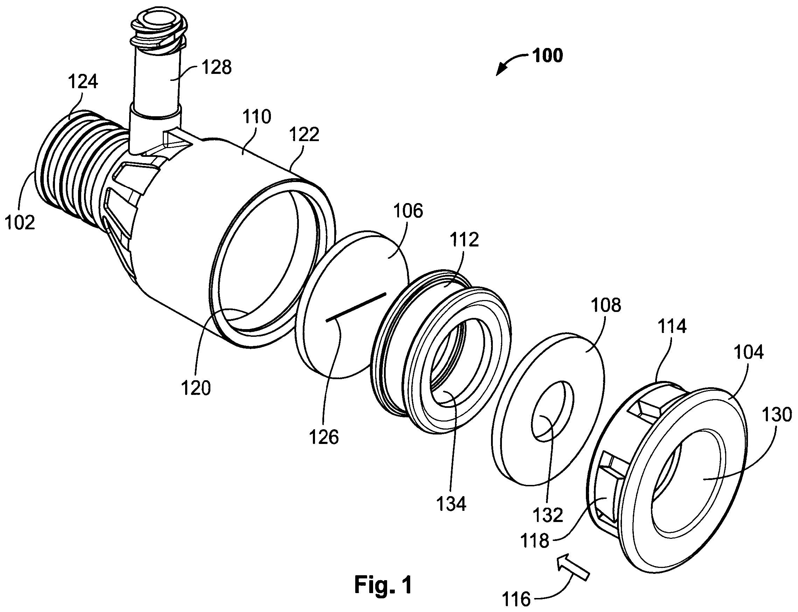

The present invention will now be explained in more detail with reference to the Figures and firstly referring to Fig. 1.

Fig. 1 shows a schematic perspective representation of a hemostasis valve assembly 100 according to the present invention. The hemostasis valve assembly has a distal end 102 with a distal opening and a proximal end 104 with a proximal opening. As used herein, the terms "proximal" and "distal" are to be taken as relative to a user using the disclosed delivery devices. "Proximal" is to be understood as relatively closer to the user and "distal" is to be understood as relatively farther away from the user.

The hemostasis valve assembly 100 comprises a distal seal 106 and the proximal seal 108. A spacer element 112 separates the distal seal 106 and the proximal seal 108 from each other. The hemostasis valve assembly 100 further comprises a hollow body 110 which encompasses the stacked arrangement formed by the two seals 106, 108 and the spacer element 112. The hollow body 110 has an essentially tubular element 122 delimiting an inner passage for inserting material, such as catheters. According to the present disclosure, the hemostasis valve assembly 100 is adapted to accommodate medical material (not shown in the Figures) having quite different radial expanses, for example, between 1 mm and 11.3 mm (3 Fr and 34 Fr), by using appropriate dimensions of the two seals 106, 108 and the spacer element 112. The outer dimensions of the hollow body 110, which is also called "hub" in the following, remain the same for all catheter sizes. At the distal end 102, the hollow body has a connector 124 to be connected to a vascular catheter (not shown in the drawings) for insertion into a patient.

Optionally, the hollow body 110 may comprise one or more tapping ports 128. The tapping port 128 may be used to extract bodily fluids or introduce substances into the patient's vessel, or to provide flushing of the inserted medical material.

According to the present invention, a ring shaped end cap 114 is provided which secures the distal seal 106, the spacer element 112, and the proximal seal 108 inside the hollow body 110. For this purpose, the end cap 114 is inserted into the hollow body 110 in a direction as indicated by the arrow 116. The end cap 114 provides a lumen 130 for the passage of the medical material.

Snap-fit hooks 118 are arranged at the end cap 114 for engaging with a locking shoulder 120 arranged at the hollow body 110. Four snap-fit hooks 118 are distributed evenly around the circumference of the end cap 114. However, it is clear for a person skilled in the art that any number of snap-fit hooks or also a continuous ring can be provided. Other fixing means, such as press fit, welding, or gluing, may of course also be used.

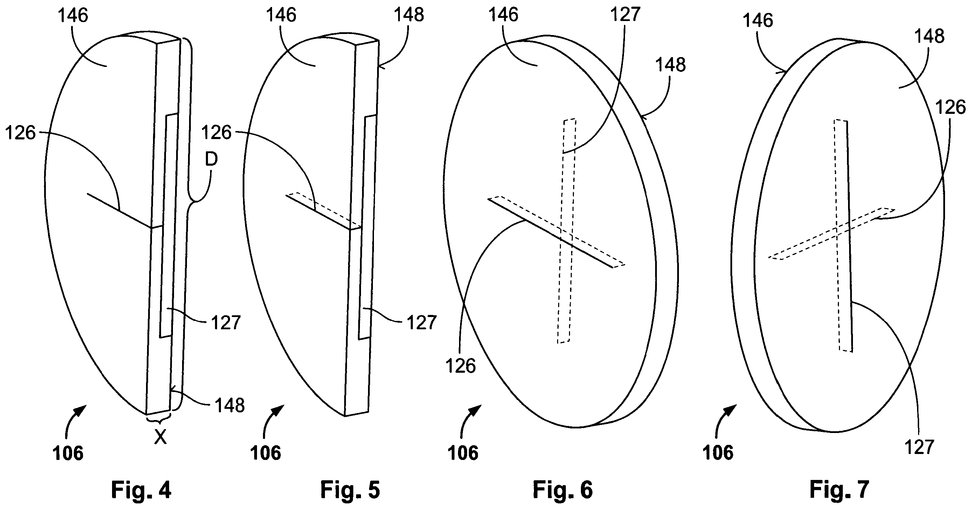

The distal seal 106 is formed as a slit seal as will be explained in more detail with reference to Figures 4 to 7. Two slits 126 are cut into an elastic material, one from each surface, extending approximately 50% into the thickness of the seal 106. The two slits 126 include an angle of e. g. 90° with each other. Such a seal design allows a particularly safe sealing already around thin inserted material, such as a guide wire, but also accommodates for high diameter material, such as 34 Fr catheters.

The proximal seal 108 is formed as a planar elastic ring with a central opening 132. The opening 132 can be chosen to match the catheter's outer diameter so as to seal and support the catheter during operation.

The modular set up shown in Fig. 1 has the advantage that the parts used for the finally assembled product can be chosen to match a wide range of outer diameters of medical material. In particular, the lumen 130 of the end cap 114, the diameter of the opening 132 of the proximal seal 108, the lumen 134 of the spacer element 112, and (optionally) the length of the slit 126 are adapted to the envisaged catheter dimensions. The hollow body 110, which provides the fluidic connector 124 and defines the outer dimensions of the hemostasis valve assembly 100, may remain the same for all possible catheter sizes.

It could be shown that by arranging the ring shaped seal and the slit seal in this sequence, i. e. with the valve functionality of the slit seal at the distal side 102, a safe sealing against the leaking of blood can be achieved, at the same time allowing for an accurate radial guidance and support of the inserted medical material, and for low insertion and retraction forces. According to the present disclosure, the valve function is not actuated by an additional external actuator but by the inserted medical material itself. This greatly simplifies the mechanical set-up of the hemostasis valve assembly. Furthermore, the distal seal 106 is capable of returning to a closed position after retraction of the medical material.

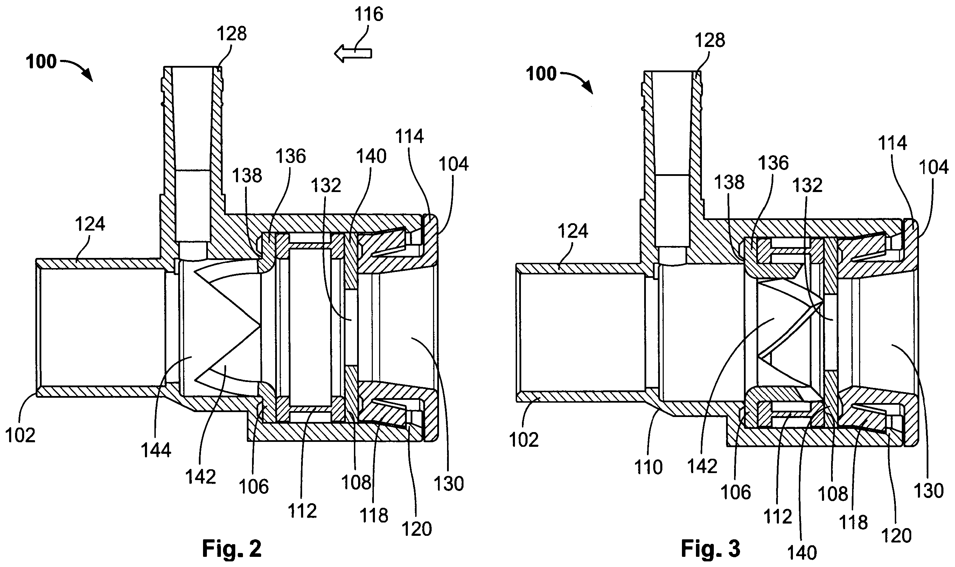

Fig. 2 and 3 schematically illustrate the deflection of the distal seal 106 during insertion (Fig. 2) and retraction (Fig. 3) of a catheter (not shown in the Figures). As can be seen from these sectional views, the distal seal 106 has a peripheral region 136 which is compressed between a retaining region 138 at the hollow body 110 and the spacer element 112. The proximal seal 108 has a second peripheral region 140, which is compressed between the spacer element 112 and the end cap 114. The axial compression forces are generated by the locking between the end cap 114 and the hollow body 110.

Turning to Fig. 2, it can be seen that a catheter that was inserted in the direction of arrow 116, displaces four segments 142 of the distal seal 106. The hollow body 110 accommodates the deflected segments 142 within a chamber 144. As will become more apparent from Fig. 4 to 7, the segments 142 are formed by at least partly rupturing the areas weakened by the slits 126 when the medical material is inserted. Advantageously, its tearing only happens to such an extent that a safe sealing to the medical material remains intact.

On the other hand, as can be seen in Fig. 3, the spacer element 112 has a recess which provides enough axial space for accommodating the segments 142 during retraction of the catheter in a direction opposite to the insertion direction 116. Advantageously, the distal seal 106 is formed from a resilient material (e. g. silicone) and the segments 142 return into the sealed position after the medical material has been completely retracted.

Figures 4 to 7 show several representations of the distal seal 106. When first turning to the sectional view of Fig. 4, the distal seal 106 has a planar disk shape with a circular outline (diameter D) and a thickness X. It is of course clear for a person skilled in the art that any other suitable outline (polygonal, oval, etc.) may also be chosen if necessary to fit the inner cross-sectional outline of the hollow body 110.

According to the present disclosure, incisions, in particular a first slit 126 and a second slit 127, are cut into the elastic material. The first slit 126 is cut from the first surface 146 to approximately X/2 (or slightly deeper). The second slit 127 is cut from the second surface 148 to approximately X/2 (or slightly deeper). The first slit 126 and the second slit 127 preferably intersect in the center of the distal seal 106 at an angle of approximately 90°. Other angles are of course also possible, but an angle of 90° leads to a particularly symmetric force distribution exerted on the inserted medical material.

Fig. 5 to 7 show further representations of the distal seal 106.

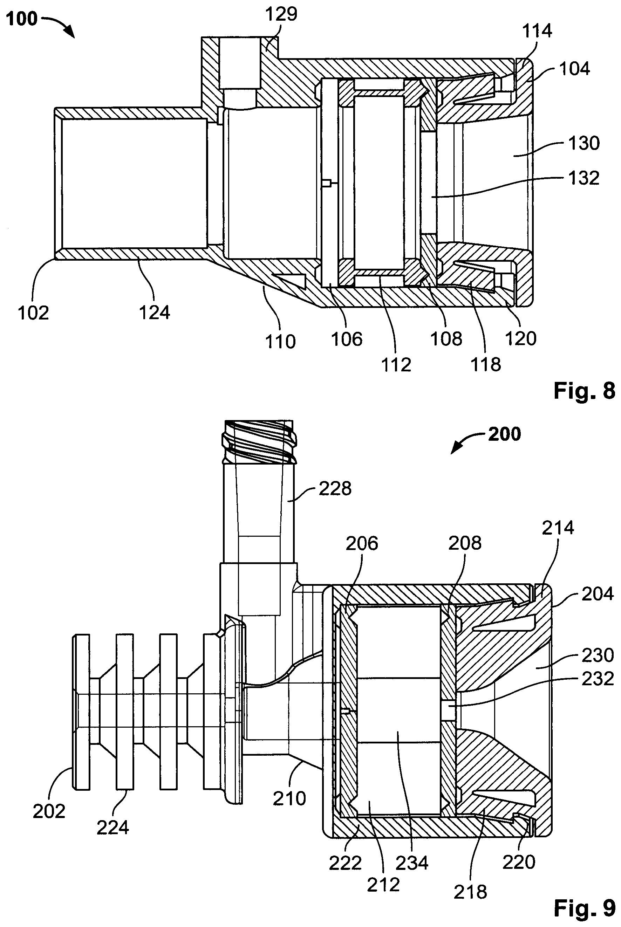

According to the present invention, before the first use the distal seal 106 only has a very small central opening at the intersection of the two incisions 126, 127, which is shut when idle (see for instance Fig. 8 below). Thus, when a thin guide wire is inserted as the first medical material during use, a tight sealing fit can be achieved around the guide wire. Only when medical material with larger diameters is inserted, the seal 106 partly ruptures along the weakened regions underlying each slit 126, 127, allowing the passage of the medical material, but still providing a tight sealing fit around the outer surface of the medical material.

Furthermore, as shown in Fig. 8, the tapping port 129 may also be configured as a flush port for bonded flush tube assemblies instead of the LUER screwed connector configuration shown in Fig. 1 to 3.

Turning now to Fig. 9, a further example of a hemostasis valve assembly 200 is shown. The valve assembly 200 is particularly adapted to receive and guide small diameter catheters. Consequently, the spacer element 212 has a smaller lumen 234 than the lumen 134 described above. Moreover, the end cap 214 has a funnel shaped lumen 230 which facilitates feeding in very fragile catheters with small diameters. The proximal seal 208 has a reduced diameter opening 232 which is small enough to seal the catheter once it is introduced into the hemostasis valve assembly 200.

The distal seal 206 is shown in the idle closed position. The distal seal 206 may be formed identical as the seals 106 explained above, or may be adapted regarding the length and depth of the incisions to the smaller diameter medical material that is inserted therethrough. Apart from the smaller diameter of the openings, all other components have analogous functionality as explained above for the hemostasis valve assembly 100 according to the first embodiment.

In summary, the present disclosure effectively solves the problem of sealing large diameter 17Fr + catheters from blood loss during procedures. The internal construction is improved to allow smooth and low force insertion and withdrawal of the device catheter.

### REFERENCE NUMERALS

| **Reference Numeral** | **Description** |
|---|---|
| 100, 200 | Hemostasis valve assembly |
| 102, 202 | Distal end of hemostasis valve assembly |
| 104, 204 | Proximal end of hemostasis valve assembly |
| 106, 206 | Distal seal |
| 108, 208 | Proximal seal |
| 110, 210 | Hollow body |
| 112, 212 | Spacer element |
| 114, 214 | End cap |
| 116 | Insertion direction |
| 118, 218 | Snap-fit hook |
| 120, 220 | Locking shoulder |
| 122, 222 | Tubular element |
| 124, 224 | Fluidic connector |
| 126 | (First) slit |
| 127 | Second slit |
| 128, 228, 129 | Tapping port |
| 130, 230 | Lumen of end cap |
| 132, 232 | Opening of proximal seal |
| 134, 234 | Lumen of spacer element |
| 136 | Peripheral region of distal seal |
| 138 | Retaining region |
| 140 | Peripheral region of proximal seal |
| 142 | Segments of distal seal |
| 144 | Chamber |
| 146 | First surface |
| 148 | Second surface |

## Claims

1. Hemostasis valve assembly (100) comprising:
a hollow body (110) delimiting an inner passage for inserting a medical material extending between a proximal opening (104) and a distal opening (102) of the hollow body (110),
a distal seal (106) arranged at the distal opening (102) of the hollow body (110) and comprising an elastic valve, which is sealed when idle,
a proximal seal (108) arranged at the proximal opening (104) of the hollow body (110),
a spacer element (112), which is arranged between the distal seal (106) and the proximal seal (108), and
an end cap (114), wherein the end cap (114) has fixing means (118) engaging with support means (120) provided at the hollow body (110), so that the stacked assembly comprising the distal seal (106), the spacer element (112), and the proximal seal (108) is fixed inside the hollow body (110),
and **characterized in that** the distal seal (106) further comprises an elastic planar seal having a first radial slit (126) extending through approximately half of the total axial thickness, and a second radial slit (127) extending through the remaining half of the total axial thickness, wherein the first and the second slit are arranged to include a radial angle with each other, so that segments (142) are formed by at least partly rupturing the areas weakened by the first radial slit (126) and the second radial slit (127) when the medical material is inserted, wherein the proximal seal (108) comprises a ring shaped elastic planar seal with an inner opening (132) delimiting a through passage aperture for receiving the medical material therethrough.

2. Hemostasis valve assembly according to claim 1, wherein the fixing means (118) is attached to the support means (120) in a static manner.

3. Hemostasis valve assembly according to claim 2, wherein the fixing means (118) comprises at least one snap-fit protrusion, and wherein the support means (120) comprises at least one corresponding snap-fit recess into which the snap-fit protrusion engages.

4. Hemostasis valve assembly according to claim 1, wherein the first and the second radial slit (126, 127) are orthogonal to each other.

5. Hemostasis valve assembly according to claim 1, wherein the inner opening (132) has a diameter which is smaller than an inner width of the spacer element (112).

6. Hemostasis valve assembly according to one of the preceding claims, wherein the hollow body (110) further comprises transverse tapping means (128, 129) for inserting a fluid into the inner passage, and wherein the tapping means is arranged on a distal side of the distal seal (106).

7. Hemostasis valve assembly according to one of the preceding claims, wherein the end cap (114) has an essentially tubular shape and is at least partly encompassed by at least a part of the hollow body (110).

8. Hemostasis valve assembly according to one of the preceding claims, wherein the distal seal (106) has a first peripheral region (136) which is compressed between a retaining region (138) at the hollow body (110) and the spacer element (112), and wherein the proximal seal (108) has a second peripheral region (140) which is compressed between the spacer element (112) and the end cap (114), and wherein axial compression forces are generated by the locking between the end cap (114) and the hollow body (110).

9. Method for assembling a hemostasis valve (100), the method comprising the following steps:
providing a hollow body (110) delimiting an inner passage for inserting a medical material extending between a proximal opening (104) and a distal opening (102) of the hollow body (110),
in an axial direction (116), inserting a distal seal (106) to be arranged at the distal opening of the hollow body (110), the distal seal comprising an elastic valve, which is sealed when idle,
inserting a spacer element (112) to be in contact with the distal seal (106),
inserting a proximal seal (108) to be arranged at the proximal opening of the hollow body (110), so that the spacer element (112) is arranged between the distal seal (106) and the proximal seal (108),
mounting an end cap (114), wherein the end cap (114) has fixing means (118) engaging with support means (120) provided at the hollow body (110), so that the stacked assembly formed by the distal seal (106), the spacer element (112), and the proximal seal (108) is fixed inside the hollow body (110),
and **characterized in that** the distal seal (106) further comprises an elastic planar seal having a first radial slit (126) extending through approximately half of the total axial thickness, and a second radial slit (127) extending through the remaining half of the total axial thickness, wherein the first and the second slit are arranged to include a radial angle with each other,
wherein segments (142) are formed by at least partly rupturing the areas weakened by the first radial slit (126) and the second radial slit (127) when the medical material is inserted,
wherein the proximal seal (106) comprises a ring shaped elastic planar seal with an inner opening (132) delimiting a through passage aperture for receiving the medical material therethrough.

10. Method according to claim 9, wherein at least one of the hollow body (110), the spacer element (112), and the end cap (114) is manufactured by 3D printing.

11. Method according to claim 9 or 10, wherein the end cap (114) is mounted at the hollow body (110) in a static manner by means of a snap-fit connection.

12. Method according to one of the claims 9 to 11, wherein the distal seal (106) and the proximal seal (108) are fabricated from an elastic material.

13. Method according to claim 12, wherein, during the step of mounting the end cap (114), a first peripheral region (136) of the distal seal (106) is compressed between a retaining region (138) at the hollow body (110) and the spacer element (112), and a second peripheral region (140) of the proximal seal (108) is compressed between the spacer element (112) and the end cap (114), and wherein axial compression forces are maintained by the locking between the end cap (114) and the hollow body (110).

## Patentansprüche

1. Hämostaseventilvorrichtung (100), umfassend:
einen Hohlkörper (110), der einen inneren Durchgang zum Einführen eines medizinischen Materials, der sich zwischen einer proximalen Öffnung (104) und einer distalen Öffnung (102) des Hohlkörpers (110) erstreckt, begrenzt
eine distale Dichtung (106), die an der distalen Öffnung (102) des Hohlkörpers (110) angeordnet ist und ein elastisches Ventil umfasst, das im Ruhezustand abgedichtet ist,
eine proximale Dichtung (108), die an der proximalen Öffnung (104) des Hohlkörpers (110) angeordnet ist,
ein Abstandselement (112), das zwischen der distalen Dichtung (106) und der proximalen Dichtung (108) angeordnet ist, und
eine Endkappe (114), wobei die Endkappe (114) Befestigungsmittel (118) aufweist, die mit am Hohlkörper (110) vorgesehenen Stützen (120) in Eingriff stehen, so dass die gestapelte Anordnung, die die distale Dichtung (106), das Abstandselement (112) und die proximale Dichtung (108) umfasst, innerhalb des Hohlkörpers (110) fixiert ist,
und **dadurch gekennzeichnet, dass** die distale Dichtung (106) ferner eine elastische ebene Dichtung mit einem ersten radialen Schlitz (126), der sich über etwa die Hälfte der gesamten axialen Dicke erstreckt, und einem zweiten radialen Schlitz (127), der sich über die verbleibende Hälfte der gesamten axialen Dicke erstreckt, wobei der erste und der zweite Schlitz so angeordnet sind, dass sie einen radialen Winkel zueinander bilden,
sodass Segmente (142) gebildet werden, indem die durch den ersten radialen Schlitz (126) und den zweiten radialen Schlitz (127) geschwächten Bereiche beim Einführen des medizinischen Materials zumindest teilweise aufgebrochen werden,
wobei die proximale Dichtung (108) eine ringförmige elastische flache Dichtung mit einer inneren Öffnung (132) umfasst, die eine Durchgangsöffnung zum Aufnehmen des medizinischen Materials durch diese begrenzt.

2. Hämostaseventilvorrichtung nach Anspruch 1, wobei das Befestigungsmittel (118) statisch an der Stütze (120) angebracht ist.

3. Hämostaseventilvorrichtung gemäß Anspruch 2, wobei das Befestigungsmittel (118) mindestens einen Einrastvorsprung umfasst und wobei das Stützmittel (120) mindestens eine entsprechende Einrastvertiefung umfasst, in die der Einrastvorsprung eingreift.

4. Hämostaseventilvorrichtung nach Anspruch 1, wobei der erste und der zweite radiale Schlitz (126, 127) orthogonal zueinander stehen.

5. Hämostaseventilvorrichtung nach Anspruch 1, wobei die innere Öffnung (132) einen Durchmesser aufweist, der kleiner ist als eine innere Breite des Abstandselements (112).

6. Hämostaseventilvorrichtung nach einem der vorstehenden Ansprüche, wobei der Hohlkörper (110) ferner eine quer verlaufende Entnahmestelle (128, 129) zum Einführen eines Fluids in den inneren Durchgang umfasst und wobei die Entnahmestelle an einer distalen Seite der distalen Dichtung (106) angeordnet ist.

7. Hämostaseventilvorrichtung nach einem der vorstehenden Ansprüche, wobei die Endkappe (114) eine im Wesentlichen röhrenförmige Gestalt aufweist und zumindest teilweise von mindestens einem Teil des Hohlkörpers (110) umschlossen ist.

8. Hämostaseventilvorrichtung nach einem der vorstehenden Ansprüche, wobei die distale Dichtung (106) einen ersten Umfangsbereich (136) aufweist, der zwischen einem Haltebereich (138) am Hohlkörper (110) und dem Abstandselement (112) zusammengedrückt ist, und wobei die proximale Dichtung (108) einen zweiten Umfangsbereich (140) aufweist, der zwischen dem Abstandselement (112) und der Endkappe (114) zusammengedrückt ist, und wobei durch die Verriegelung zwischen der Endkappe (114) und dem Hohlkörper (110) axiale Kompressionskräfte erzeugt werden.

9. Verfahren zum Zusammenbau eines Hämostaseventils (100), wobei das Verfahren die folgenden Schritte umfasst:
Bereitstellen eines Hohlkörpers (110), der einen inneren Durchgang zum Einführen eines medizinischen Materials begrenzt, der sich zwischen einer proximalen Öffnung (104) und einer distalen Öffnung (102) des Hohlkörpers (110) erstreckt,
Einführen einer distalen Dichtung (106) in axialer Richtung (116), die an der distalen Öffnung des Hohlkörpers (110) angeordnet werden soll, wobei die distale Dichtung ein elastisches Ventil umfasst, das im Ruhezustand abgedichtet ist,
Einführen eines Abstandselements (112), das mit der distalen Dichtung (106) in Kontakt steht,
Einführen einer proximalen Dichtung (108), die an der proximalen Öffnung des Hohlkörpers (110) angeordnet werden soll, so dass das Abstandselement (112) zwischen der distalen Dichtung (106) und der proximalen Dichtung (108) angeordnet ist,
Anbringen einer Endkappe (114), wobei die Endkappe (114) Befestigungsmittel (118) aufweist, die mit am Hohlkörper (110) vorgesehenen Stützen (120) in Eingriff stehen, sodass die durch die distale Dichtung (106), das Abstandselement (112) und die proximale Dichtung (108) gebildete gestapelte Anordnung innerhalb des Hohlkörpers (110) fixiert ist,
und **dadurch gekennzeichnet, dass** die distale Dichtung (106) ferner eine elastische ebene Dichtung mit einem ersten radialen Schlitz (126), der sich über etwa die Hälfte der gesamten axialen Dicke erstreckt, und einem zweiten radialen Schlitz (127), der sich über die verbleibende Hälfte der gesamten axialen Dicke erstreckt, wobei der erste und der zweite Schlitz so angeordnet sind, dass sie einen radialen Winkel zueinander bilden,
wobei Segmente (142) gebildet werden, indem die durch den ersten radialen Schlitz (126) und den zweiten radialen Schlitz (127) geschwächten Bereiche beim Einführen des medizinischen Materials zumindest teilweise aufgebrochen werden,
wobei die proximale Dichtung (106) eine ringförmige elastische flache Dichtung mit einer inneren Öffnung (132) umfasst, die eine Durchgangsöffnung zum Aufnehmen des medizinischen Materials durch diese begrenzt.

10. Verfahren nach Anspruch 9, wobei mindestens eines von Hohlkörper (110), Abstandselement (112) und Endkappe (114) mittels 3D-Druck hergestellt wird.

11. Verfahren nach Anspruch 9 oder 10, wobei die Endkappe (114) mittels einer Einrastverbindung statisch am Hohlkörper (110) befestigt ist.

12. Verfahren nach einem der Ansprüche 9 bis 11, wobei die distale Dichtung (106) und die proximale Dichtung (108) aus einem elastischen Material hergestellt sind.

13. Verfahren nach Anspruch 12, wobei während des Schritts des Anbringen der Endkappe (114) ein erster Umfangsbereich (136) der distalen Dichtung (106) zwischen einem Haltebereich (138) am Hohlkörper (110) und dem Abstandselement (112) zusammengedrückt wird und ein zweiter Umfangsbereich (140) der proximalen Dichtung (108) zwischen dem Abstandselement (112) und der Endkappe (114) zusammengedrückt wird, und wobei die axialen Kompressionskräfte durch die Verriegelung zwischen der Endkappe (114) und dem Hohlkörper (110) aufrechterhalten werden.

## Revendications

1. Ensemble de valve hémostatique (100) comprenant :
un corps creux (110) délimitant un passage intérieur pour insérer un matériel médical s'étendant entre une ouverture proximale (104) et une ouverture distale (102) du corps creux (110),
un joint étanche distal (106) disposé au niveau de l'ouverture distale (102) du corps creux (110) et comprenant une valve élastique, qui est étanchéifiée au repos,
un joint étanche proximal (108) disposé au niveau de l'ouverture proximale (104) du corps creux (110),
un élément espaceur (112), qui est disposé entre le joint étanche distal (106) et le joint étanche proximal (108), et
un bouchon d'extrémité (114), dans lequel le bouchon d'extrémité (114) a des moyens de fixation (118) qui viennent en prise avec des moyens de support (120) agencés au niveau du corps creux (110), de sorte que l'ensemble empilé comprenant le joint étanche distal (106), l'élément espaceur (112) et le joint étanche proximal (108) est fixé à l'intérieur du corps creux (110),
et caractérisé en ce sens que le joint étanche distal (106) comprend en outre un joint étanche planaire élastique ayant une première fente radiale (126) s'étendant sur approximativement la moitié de l'épaisseur axiale totale, et une seconde fente radiale (127) s'étendant sur la moitié restante de l'épaisseur axiale totale, dans lequel la première et la seconde fente sont disposées pour inclure un angle radial l'une par rapport à l'autre,
de sorte que des segments (142) sont formés en rompant au moins partiellement les zones affaiblies par la première fente radiale (126) et la seconde fente radiale (127) lors de l'insertion du matériel médical, dans lequel le joint étanche proximal (108) comprend un joint étanche planaire élastique en forme d'anneau avec une ouverture intérieure (132) délimitant un orifice de passage traversant pour recevoir le matériel médical à travers celui-ci.

2. Ensemble de valve hémostatique selon la revendication 1, dans lequel les moyens de fixation (118) sont attachés de manière statique aux moyens de support (120).

3. Ensemble de valve hémostatique selon la revendication 2, dans lequel les moyens de fixation (118) comprennent au moins une saillie encliquetable, et dans lequel les moyens de support (120) comprennent au moins une cavité encliquetable correspondante dans laquelle la saillie encliquetable vient en prise.

4. Ensemble de valve hémostatique selon la revendication 1, dans lequel la première et la seconde fente radiale (126, 127) sont orthogonales l'une à l'autre.

5. Ensemble de valve hémostatique selon la revendication 1, dans lequel l'ouverture intérieure (132) a un diamètre qui est inférieur à une largeur intérieure de l'élément espaceur (112).

6. Ensemble de valve hémostatique selon l'une des revendications précédentes, dans lequel le corps creux (110) comprend en outre des moyens de soutirage transversaux (128, 129) pour insérer un fluide dans le passage intérieur, et dans lequel les moyens de soutirage sont disposés sur un côté distal du joint étanche distal (106).

7. Ensemble de valve hémostatique selon l'une des revendications précédentes, dans lequel le bouchon d'extrémité (114) a une forme essentiellement tubulaire et est au moins partiellement englobé par au moins une partie du corps creux (110).

8. Ensemble de valve hémostatique selon l'une des revendications précédentes, dans lequel le joint étanche distal (106) a une première région périphérique (136) qui est comprimée entre une région de retenue (138) au niveau du corps creux (110) et l'élément espaceur (112), et dans lequel le joint étanche proximal (108) a une seconde région périphérique (140) qui est comprimée entre l'élément espaceur (112) et le bouchon d'extrémité (114), et dans lequel des forces de compression axiale sont générées par le verrouillage entre le bouchon d'extrémité (114) et le corps creux (110).

9. Procédé pour assembler une valve hémostatique (100), le procédé comprenant les étapes consistant à :
fournir un corps creux (110) délimitant un passage intérieur pour insérer un matériel médical s'étendant entre une ouverture proximale (104) et une ouverture distale (102) du corps creux (110),
dans une direction axiale (116), insérer un joint étanche distal (106) à disposer au niveau de l'ouverture distale du corps creux (110), le joint étanche distal comprenant une valve élastique, qui est étanchéifiée au repos,
insérer un élément espaceur (112) pour être en contact avec le joint étanche distal (106),
insérer un joint étanche proximal (108) à disposer au niveau de l'ouverture proximale du corps creux (110), de sorte que l'élément espaceur (112) est disposé entre le joint étanche distal (106) et le joint étanche proximal (108),
monter un bouchon d'extrémité (114), dans lequel le bouchon d'extrémité (114) a des moyens de fixation (118) qui viennent en prise avec des moyens de support (120) agencés au niveau du corps creux (110), de sorte que l'ensemble empilé formé par le joint étanche distal (106), l'élément espaceur (112) et le joint étanche proximal (108) est fixé à l'intérieur du corps creux (110),
et **caractérisé en ce que** le joint étanche distal (106) comprend en outre un joint étanche planaire élastique ayant une première fente radiale (126) s'étendant sur approximativement la moitié de l'épaisseur axiale totale, et une seconde fente radiale (127) s'étendant sur la moitié restante de l'épaisseur axiale totale, dans lequel la première et la seconde fente sont disposées pour inclure un angle radial l'une par rapport à l'autre,
dans lequel des segments (142) sont formés en rompant au moins partiellement les zones affaiblies par la première fente radiale (126) et la seconde fente radiale (127) lors de l'insertion du matériel médical,
dans lequel le joint étanche proximal (108) comprend un joint étanche planaire élastique en forme d'anneau avec une ouverture intérieure (132) délimitant un orifice de passage traversant pour recevoir le matériel médical à travers celui-ci.

10. Procédé selon la revendication 9, dans lequel au moins l'un parmi le corps creux (110), l'élément espaceur (112) et le bouchon d'extrémité (114) est fabriqué par impression 3D.

11. Procédé selon la revendication 9 ou 10, dans lequel le bouchon d'extrémité (114) est monté au niveau du corps creux (110) de manière statique au moyen d'une connexion encliquetable.

12. Procédé selon l'une des revendications 9 à 11, dans lequel le joint étanche distal (106) et le joint étanche proximal (108) sont fabriqués à partir d'un matériau élastique.

13. Procédé selon la revendication 12, dans lequel, lors de l'étape de montage du bouchon d'extrémité (114), une première région périphérique (136) du joint étanche distal (106) est comprimée entre une région de retenue (138) au niveau du corps creux (110) et l'élément espaceur (112), et une seconde région périphérique (140) du joint étanche proximal (108) est comprimée entre l'élément espaceur (112) et le bouchon d'extrémité (114), et dans lequel des forces de compression axiale sont maintenues par le verrouillage entre le bouchon d'extrémité (114) et le corps creux (110).
